(19)

**Europäisches Patentamt**

**European Patent Office**

**Office européen des brevets**

(11) **EP 4 339 610 A1**

(12) **EUROPEAN PATENT APPLICATION**
published in accordance with Art. 153(4) EPC

(43) Date of publication:
**20.03.2024 Bulletin 2024/12**

(21) Application number: **23730690.7**

(22) Date of filing: **19.04.2023**

(51) International Patent Classification (IPC):
**G01N 33/04** *(2006.01)*

(52) Cooperative Patent Classification (CPC):
Y02P 90/30

(86) International application number:
**PCT/CN2023/089177**

(87) International publication number:
**WO 2024/021694 (01.02.2024 Gazette 2024/05)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **29.07.2022 CN 202210902419**

(71) Applicant: **Beijing Sanyuan Foods Co., Ltd.
Beijing 100163 (CN)**

(72) Inventors:
• **CHEN, Lijun
Beijing 100163 (CN)**
• **LIU, Qian
Beijing 100163 (CN)**

• **QIAO, Weicang
Beijing 100163 (CN)**
• **LIU, Yan
Beijing 100163 (CN)**
• **ZHAO, Junying
Beijing 100163 (CN)**
• **LIU, Bin
Beijing 100163 (CN)**
• **LIU, Yanpin
Beijing 100163 (CN)**
• **LIU, Yan
Beijing 100163 (CN)**
• **CHEN, Jingyao
Beijing 100163 (CN)**

(74) Representative: **LLR
2, rue Jean Lantier
75001 Paris (FR)**

(54) **METHOD FOR EVALUATING MILK PRODUCTS ACCORDING TO LIPIDIC COMPONENTS**

(57) The present application relates to a method for evaluating dairy products based on lipid components. The method is carried out according to the following steps: (1) establishing local breast milk glyceride database; (2) collecting and pretreating to-be-evaluated dairy product samples; (3) measuring the contents of characteristic glycerides in the to-be-evaluated dairy product samples; and (4) calculating the scores G of the to-be-evaluated dairy product samples based on the following formula:

$$G=100-\Sigma\left(100\times \frac{H_i}{H_t} \times \frac{\mid H_{ih/l}-A_i \mid}{A_i}\right)$$

$$H_{ih/l}=\begin{cases} 1.3H_i, & A_i > 1.3H_i \\ A_i, & 1.3H_i \geq A_i \geq 0.7H_i \\ 0.7H_i, & A_i < 0.7H_i \end{cases}$$

; in the formula, wherein, $H_i$ is the content of characteristic glyceride i in breast milk, $H_t$ is the content of total glycerides in breast milk, and $A_i$ is the content of the characteristic glyceride i in the sample. The present application evaluates infant formula and raw and auxiliary materials in multiple dimensions from two aspects of composition and content of glyceride, thereby objectively evaluating the nutritional level of dairy products or raw and auxiliary materials in a molecular level, which has certain guiding significance for subsequent simulation of breast milk.

FIG. 1

**Description**

**Cross-Reference to Related Application**

The present application claims priority to Chinese Patent Application No. 202210902419.0 filed with the China National Intellectual Property Administration on July 29, 2022 and entitled "METHOD FOR EVALUATING DAIRY PRODUCTS BASED ON LIPID COMPONENTS", the disclosure of which is incorporated by reference herein in its entirety as part of the present application.

**Technical Field**

The present application relates to the field of dairy products, particularly to a method for evaluating dairy products based on lipid components.

**Background Art**

Breast milk provided by a mother who is healthy and good in nutrition provides ideal food comprising essential nutrients, growth factors and immune components, which is crucial to short and long-term development of infants. However, in cases where breastfeeding cannot be selected or breast milk is unavailable, infant formula becomes primary food for infants. Breast milk has always been a gold standard for infant formula simulation. Glyceride is a main component of breast milk and infant formula which has very important nutrient characteristics, for example providing about 50% of energy for rapid growth of infants, providing essential fatty acids for brain and visual development of infants, and facilitating immune system development. Glyceride is an important component of body tissues and many organs, and is a good carrier for the absorption of fat-soluble vitamins (A, D, E and K). These characteristics are closely related to compositions and contents of fatty acids. In addition, the compositions and contents of lipids in breast milk are affected by diet, lactation, genes and regions, and the compositions and contents of lipids in infant formula are mainly affected by raw and auxiliary materials and processing technologies. Throughout the history of lipid development of infant formula, the application of vegetable oils, simulation of structural lipids, and supplementation of functional fatty acids are involved when the infant formula simulates breast milk lipids. That is, a more scientific and reasonable matching is achieved in the aspects of the composition, content and structure of glyceride. However, there are few reports on evaluation methods for the quality of infant formula simulation. In China, the vast majority of infant formulas are based on fresh milk, and the lipid portion is made from the fat in milk combined with vegetable oil to simulate breast milk lipids. The compositions and contents of lipids in infant formula directly depend on raw and auxiliary materials used in the processing of formula. However, there are no reports on the evaluation methods for lipid quality of raw and auxiliary materials.

At present, in most cases, methods such as chromatography and mass spectrometry are used for analyzing the quality of lipids in infant formula and its raw and auxiliary materials, and illustrating the difference of lipids between infant formula and its raw and auxiliary materials and breast milk by comparative analysis. The previous evaluation techniques mostly conduct evaluation and comparison from the levels of fatty acids, lipid subclasses, or certain glycerides, without fully considering the compositions and contents of lipids and without considering the weights of different species of glycerides.

In view of this, the present application is hereby proposed.

**Summary of the Invention**

The objective of the present application is to provide a method for comprehensive, detailed and multi-dimensional evaluation of dairy products using breast milk glyceride database as a gold standard.

To achieve the above objective, the present application provides a method for evaluating dairy products based on lipid components, the method being carried out according to the following steps:

(1) Establishing local breast milk glyceride database;
(2) Collecting and pretreating to-be-evaluated dairy product samples;
(3) Measuring the contents of characteristic glycerides in the to-be-evaluated dairy product samples; and
(4) Calculating the scores G of the to-be-evaluated dairy product samples based on the following formula:

$$G = 100 - \Sigma\left(100 \times \frac{H_i}{H_t} \times \frac{|H_{ih/l} - A_i|}{A_i}\right);$$

$$H_{ih/l} = \begin{cases} 1.3H_i, & A_i > 1.3H_i \\ A_i, & 1.3H_i \geq A_i \geq 0.7H_i \\ 0.7H_i, & A_i < 0.7H_i \end{cases};$$

In the formula,

Wherein, $H_i$ is the content of characteristic glyceride i in breast milk, $H_t$ is the content of total glycerides in breast milk, and $A_i$ is the content of the characteristic glyceride i in the sample. Preferably or optionally, the pretreating is carried out according to the following steps:

diluting a sample with a redissolving solution so that the concentration of the sample is consistent with that of breast milk, adding an internal standard to the sample, then adding ultrapure water, methanol and dichloromethane, mixing the above materials, and then adding ultrapure water and dichloromethane, centrifuging the mixed solution to separate an organic phase from an aqueous phase, adding ultrapure water, methanol and dichloromethane into the organic phase, evenly mixing, then centrifuging the mixed solution to separate an organic phase from an aqueous phase, merging the aqueous phases obtained after the two centrifugations, adding dichloromethane, mixing and centrifuging the mixed solution to separate an organic phase from an aqueous phase; and merging the organic phases obtained after the last two centrifugations, blowing the organic phases to be dried with nitrogen, and then redissolving the dried organic phases with the redissolving solution.

Preferably or optionally, the redissolving solution used in the pretreating is a methanol-dichloromethane solution containing 0.5-2 mM ammonium acetate, and a volume ratio of methanol to dichloromethane in the redissolving solution is 1:1-2; preferably, the redissolving solution is a methanol-dichloromethane solution containing 1 mM ammonium acetate, and a volume ratio of methanol to dichloromethane in the redissolving solution is 1:1.

Preferably or optionally, the internal standard is a d5-TAG54:3 standard product with a concentration of 3000-5000 mg/L, preferably 4000 mg/L.

Preferably or optionally, in step (3), a method for measuring the contents of characteristic glycerides is an ultra-high performance liquid chromatography tandem quadrupole time-of-flight mass spectrometry.

Preferably or optionally, the method for the ultra-high performance liquid chromatography is as follows: the chromatographic column is Phenomenex, Kinetex 2.6 $\mu$m C18 100 Å 50 × 3 mm, with an injection volume of 2 $\mu$L, a flow rate of 0.8 mL/min and a column temperature of 30 °C; mobile phase A is a water/methanol/acetonitrile (1:1:1, v/v/v) solution of 5 mM ammonium acetate, and mobile phase B is an isopropanol solution of 5 mM ammonium ethanol; an elution gradient is:

| Glyceride (Time, min) | 0.0 | 5.0 | 8.0 | 8.01 | 10.0 |
|---|---|---|---|---|---|
| A | 50% | 2% | 2% | 50% | 50% |
| B | 50% | 98% | 98% | 50% | 50% |

Preferably or optionally, the conditions of the quadrupole time-of-flight mass spectrometry are as follows: under the condition of a positive pole, a spray voltage of an ion source is 5500 V, a gas pressure of the ion source is 60 psi, a temperature of the ion source is 650 °C, a pressure of a curtain gas is 35 psi, a de-clustering voltage is 80 V, a collision energy is 10 V, and a scanning range is 50-1300 Da.

Preferably or optionally, in step (3), the characteristic glycerides comprise TAG52:3 (16:0-18:1-18:2), TAG52:2 (16:0-18:1-18:1), TAG52:4 (16:0-18:2-18:2), TAG54:4 (18:1-18:1-18:2), TAG54:3 (18:1-18:1-18:1), TAG50:2 (16:0-16:1-18:1), TAG54:5 (18:1-18:2-18:2), TAG54:3 (18:0-18:1-18:2), TAG50:1 (16:0-16:0-18:1), TAG52:1 (16:0-18:0-18:1), TAG48:2 (12:0-18:1-18:1), TAG48:2 (14:0-16:0-18:2), TAG46:1 (12:0-16:0-18:1), TAG50:3 (14:0-18:1-18:2), TAG46:2 (12:0-16:0-18:2), TAG48:1 (14:0-16:0-18:1), TAG48:3 (12:0-18:1-18:2), TAG44:1 (12:0-14:0-18:1), TAG54:6 (18:2-18:2-18:2), and TAG54:2 (18:0-18:1-18:1).

Preferably or optionally, the characteristic glycerides further comprise TAG58:8 (18:1-18:1-22:6), TAG58:9 (18:1-18:2-22:6), TAG58:10 (18:2-18:2-22:6), TAG56:7 (18:1-18:2-20:4), and TAG56:8 (18:2-18:2-20:4).

Preferably or optionally, the characteristic glycerides further comprise TAG 44:2 (12:0-14:0-18:2), TAG 52:5 (16:0-18:2-18:3), TAG 50:4 (14:0-18:2-18:2), TAG 44:0 (12:0-14:0-18:0), TAG 42:0 (12:0-14:0-16:0), TAG 48:4 (12:0-18:2-18:2), TAG 42:1 (12:0-12:0-18:1), TAG 40:0 (12:0-14:0-14:0), TAG 46:0 (12:0-16:0-18:0), TAG 46:3 (12:0-16:1-18:2), TAG 48:0 (14:0-16:0-18:0), TAG 56:5 (18:1-18:2-20:2), TAG 56:6 (18:1-18:2-20:3), TAG 50:0 (16:0-16:0-18:0), TAG 42:2 (12:0-12:0-18:2), TAG 38:0 (12:0-12:0-14:0), TAG 56:4 (18:1-18:2-20:1), TAG 56:3 (18:1-18:1-20:1), TAG 54:7 (18:2-18:2-18:3), TAG 40:1 (12:0-12:0-16:1), TAG 54:1 (18:0-18:0-18:1), TAG 54:1 (16:0-18:1-20:0), TAG 36:0 (12:0-12:0-12:0), DAG 34:2 (16:0-18:2), TAG 56:2 (16:0-18:1-22:1), TAG 52:0 (16:0-18:0-18:0), DAG 34:1 (16:0-18:1), TAG 56:3 (18:1-18:2-20:0), TAG 51:2 (16:0-17:1-18:1), TAG 53:2 (17:0-18:1-18:1), TAG 50:5 (14:0-18:2-18:3), TAG 58:6 (18:1-18:1-22:4), TAG 53:3 (17:0-18:1-18:2), TAG 58:7 (18:1-18:2-22:4), TAG 44:3 (12:0-14:0-18:3), DAG 36:3 (18:1-18:2), TAG 51:3 (15:0-18:1-18:2), TAG 51:1 (16:0-17:0-18:1), TAG 52:6 (16:1-18:2-18:3), TAG 58:4 (18:1-18:2-22:1), TAG 53:4 (17:1-18:1-18:2), TAG 48:5 (12:0-18:2-18:3), TAG 58:5 (18:1-18:2-22:2), DAG 36:4 (18:2-18:2), TAG 49:1 (15:0-16:0-18:1), DAG 36:2 (18:1-18:1), TAG 49:2 (15:0-16:0-18:2), TAG 58:2 (18:1-18:1-22:0), TAG 58:3 (18:1-18:2-22:0), TAG 56:1 (16:0-18:1-22:0), DAG 34:0 (16:0-18:0), TAG 47:1 (12:0-17:0-18:1), DAG 32:0 (16:0-16:0), DAG 32:1 (14:0-18:1), TAG 47:2 (12:0-17:0-18:2), TAG 45:1 (12:0-15:0-18:1), TAG 42:3 (12:0-12:0-18:3), TAG 51:4 (15:0-18:2-18:2), DAG 32:2 (14:0-18:2), TAG 53:1 (17:0-18:0-18:1), TAG 58:1 (18:0-18:1-22:0), DAG 28:0 (12:0-16:0), DAG 34:3 (16:1-18:2), DAG 36:1 (18:0-18:1), DAG 30:0 (14:0-16:0), DAG 30:2 (12:0-18:2), TAG 45:2 (12:0-15:0-18:2) and DAG 30:1 (12:0-18:1), and TAG 54:0 (16:0-18:0-20:0).

The present application evaluates infant formula and raw and auxiliary materials in multiple dimensions from two aspects of composition and content of glyceride. First, infant formula and raw materials are overall evaluated through cluster analysis; then, the reasons for differences between lipid compositions are explored by analyzing the glycerides of breast milk, infant formula, and its raw and auxiliary materials; finally, with the composition and content of glyceride molecular species in breast milk as a standard, the quality of infant formula and its raw and auxiliary materials are comprehensively evaluated by comparing the compositions and contents of glycerides in infant formula and its raw and auxiliary materials that are the same as those in breast milk.

**Brief Description of the Drawings**

FIG. 1 is a comparison graph of characteristic glyceride contents in each dairy sample in Example 2;
FIG. 2 is a result graph of intestinal tract $\alpha$-diversity of piglets fed in formula group 1 and formula group 2 at 21d in Example 3;
FIG. 3 is a result graph of intestinal tract $\beta$-diversity of piglets fed in breast milk group, formula group 1 and formula group 2 at 21d in Example 3;
FIG. 4 is a result graph of immune factors of piglets fed in breast milk group, formula group 1 and formula group 2 at 21d in Example 3;
FIG. 5 is a prevalence graph of infants aged 0-6 months fed in breast milk group, formula group 1 and formula group 2 in Example 3;
FIG. 6 is an ages & stages questionnaires (ASQ) test result graph of infants aged 4-6 months fed in breast milk group, formula group 1 and formula group 2 in Example 3; and
FIG. 7 is an intestinal flora graph of infants fed in breast milk group, formula group 1 and formula group 2 in Example 3.

**Detailed Description of the Invention**

Next, the specific embodiments of the present application will be described in detail. It should be understood that the specific embodiments described here are only for illustrating and explaining the present application but not limiting the present application.

Unless otherwise specified, experimental methods used in the following examples are all conventional methods.

Unless otherwise specified, materials and reagents used in the following examples are all commercially available.

Example 1

This example provides a method for establishing a local breast milk database. 61 healthy lying-in women from Beijing, Tangshan, Liuyang and Luoyang were selected, and the relevant physical indicators of each lying-in woman were normal, and infants were all born at term.

A total of 233 breast milk samples aged 0-6 months were traced and collected from the 61 healthy lying-in women mentioned above, and the infant development status corresponding to each breast milk sample was recorded at the same time.

The samples were pretreated before chromatography and mass spectrometry. The specific pretreatment method was as follows: the samples were diluted with a redissolving solution to a concentration that was similar to that of breast milk, wherein the redissolving solution was a methanol:dichloromethane (1: 1, v/v) solution containing 1 mM ammonium acetate. 20 $\mu$L of 4000 mg/L d5-TAG54:3 standard sample was added into 200 $\mu$L of a to-be-evaluated sample, 200 $\mu$L of ultrapure water, 2 mL of methanol and 900 $\mu$L of dichloromethane were added, the above materials were evenly mixed, then 200 $\mu$L of ultrapure water and 900 $\mu$L of dichloromethane were added, the above materials were evenly mixed, the obtained mixture was centrifuged for 15 min at 6000 rpm to separate an organic phase from an aqueous phase. 1 mL of ultrapure water, 2.2 mL of methanol and 600 μL of dichloromethane were added into the organic phase, and then the obtained mixed solution was centrifuged for 10 min at 3000 ×g to separate an organic phase from an aqueous phase. The aqueous phases were merged and then mixed with 1.8 mL of dichloromethane, the obtained mixed solution was centrifuged for 15 min at 6000 rpm, and then the organic phases obtained after the last two centrifugations were merged. The merged organic phase was blown to be dried with nitrogen, and then redissolved with 1 mL of a redissolving solution. Dilution was carried out by 1000 folds with 50 μL of a redissolving solution, and then the solution obtained after dilution was directly used for subsequent detection.

The species and contents of glycerides in the above breast milk samples were measured by using an ultra-high performance liquid chromatography/quadrupole time-of-flight mass spectrometry (UPLC/Q-TOF-MS) method and an equipped ESI electric spray ion source as well as Pearkview 2.2 and MSDIAL 3.20 data processing systems.

Specific chromatographic conditions were as follows: a chromatographic column was Phenomenex, Kinetex 2.6 μm C18 100 Å 50 × 3 mm, with an injection volume of 2 μL, a flow rate of 0.8 mL/min and a column temperature of 30 °C.

Mobile phase A was a water/methanol/acetonitrile (1:1:1, v/v/v) solution containing 5 mM ammonium acetate, and mobile phase B was an isopropanol solution containing 5 mM ethanol ammonium;

The elution gradient is shown in Table 1:

Table 1 Chromatographic elution gradient table

| Glyceride (time, min) | 0.0 | 5.0 | 8.0 | 8.01 | 10.0 |
|---|---|---|---|---|---|
| A | 50% | 2% | 2% | 50% | 50% |
| B | 50% | 98% | 98% | 50% | 50% |

The conditions of the quadrupole time-of-flight mass spectrometry were as follows: under the condition of a positive pole, a spray voltage of an ion source was 5500 V, a gas pressure of the ion source was 60 psi, a temperature of the ion source was 650 °C, a pressure of a curtain gas was 35 psi, a de-clustering voltage was 80 V, a collision energy was 10 V, and a scanning range was 50-1300 Da.

A total of 94 characteristic glycerides were measured by using chromatography and mass spectrometry mentioned above, and the above 94 glycerides were classified according to the contents and functions of each glyceride. The results are shown in Table 2.

Table 2 Content results of 94 characteristic glyerides in breast milk sample in Example 1

| Category | Glyceride type | Breast milk weight (%) |
|---|---|---|
| Main glycerides (top 20) | TAG 52:3 (16:0-18:1-18:2) | 12.35 |
| | TAG 52:2 (16:0-18:1-18:1) | 11.37 |
| | TAG 52:4 (16:0-18:2-18:2) | 5.77 |
| | TAG 54:4 (18:1-18:1-18:2) | 5.55 |
| | TAG 54:3 (18:1-18:1-18:1) | 4.75 |
| | TAG 50:2 (16:0-16:1-18:1) | 4.58 |
| | TAG 54:5 (18:1-18:2-18:2) | 4.25 |
| | TAG 54:3 (18:0-18:1-18:2) | 3.40 |
| | TAG 50:1 (16:0-16:0-18:1) | 3.34 |
| | TAG 52:1 (16:0-18:0-18:1) | 3.06 |
| | TAG48:2 (12:0-18:1-18:1) | 2.94 |
| | TAG 48:2 (14:0-16:0-18:2) | 2.89 |
| | TAG 46:1 (12:0-16:0-18:1) | 2.89 |
| | TAG 50:3 (14:0-18:1-18:2) | 2.71 |
| | TAG 46:2 (12:0-16:0-18:2) | 2.16 |
| | TAG 48:1 (14:0-16:0-18:1) | 2.12 |
| | TAG 48:3 (12:0-18:1-18:2) | 1.95 |
| | TAG 44:1 (2:0-14:0-18:1) | 1.81 |
| | TAG 54:6 (18:2-18:2-18:2) | 1.81 |
| | TAG 54:2 (18:0-18:1-18:1) | 1.36 |
| DHA-TAG | TAG 58:8 (18:1-18:1-22:6) | 0.15 |
| | TAG 58:9 (18:1-18:2-22:6) | 0.11 |
| | TAG 58:10 (18:2-18:2-22:6) | 0.04 |
| AA-TAG | TAG 56:7 (18:1-18:2-20:4) | 0.33 |
| | TAG 56:8 (18:2-18:2-20:4) | 0.13 |

(continued)

| Category | Glyceride type | Breast milk weight (%) |
|---|---|---|
| Others | TAG 44:2 (12:0-14:0-18:2) | 1.07 |
| | TAG 52:5 (16:0-18:2-18:3) | 0.96 |
| | TAG 50:4 (14:0-18:2-18:2) | 0.94 |
| | TAG 44:0 (12:0-14:0-18:0) | 0.92 |
| | TAG 42:0 (12:0-14:0-16:0) | 0.77 |
| | TAG 48:4 (12:0-18:2-18:2) | 0.75 |
| | TAG 42:1 (12:0-12:0-18:1) | 0.74 |
| | TAG 40:0 (12:0-14:0-14:0) | 0.74 |
| | TAG 46:0 (12:0-16:0-18:0) | 0.73 |
| | TAG 46:3 (12:0-16:1-18:2) | 0.60 |
| | TAG 48:0 (14:0-16:0-18:0) | 0.52 |
| | TAG 56:5 (18:1-18:2-20:2) | 0.49 |
| | TAG 56:6 (18:1-18:2-20:3) | 0.48 |
| | TAG 50:0 (16:0-16:0-18:0) | 0.47 |
| | TAG 42:2 (12:0-12:0-18:2) | 0.42 |
| | TAG 38:0 (12:0-12:0-14:0) | 0.40 |
| | TAG 56:4 (18:1-18:2-20:1) | 0.38 |
| | TAG 56:3 (18:1-18:1-20:1) | 0.33 |
| | TAG 54:7 (18:2-18:2-18:3) | 0.30 |
| | TAG 40:1 (12:0-12:0-16:1) | 0.27 |
| | TAG 54:1 (18:0-18:0-18:1) | 0.25 |
| | TAG 54:1 (16:0-18:1-20:0) | 0.24 |
| | TAG 36:0 (12:0-12:0-12:0) | 0.24 |
| | DAG 34:2 (16:0-18:2) | 0.23 |
| | TAG 56:2 (16:0-18:1-22:1) | 0.23 |
| | TAG 52:0 (16:0-18:0-18:0) | 0.22 |
| | DAG 34:1 (16:0-18:1) | 0.22 |
| | TAG 56:3 (18:1-18:2-20:0) | 0.22 |

(continued)

| Category | Glyceride type | Breast milk weight (%) |
|---|---|---|
| | TAG 51:2 (16:0-17:1-18:1) | 0.21 |
| | TAG 53:2 (17:0-18:1-18:1) | 0.21 |
| | TAG 50:5 (14:0-18:2-18:3) | 0.19 |
| | TAG 58:6 (18:1-18:1-22:4) | 0.18 |
| | TAG 53:3 (17:0-18:1-18:2) | 0.18 |
| | TAG 58:7 (18:1-18:2-22:4) | 0.18 |
| | TAG 44:3 (12:0-14:0-18:3) | 0.16 |
| | DAG 36:3 (18:1-18:2) | 0.16 |
| | TAG 51:3 (15:0-18:1-18:2) | 0.15 |
| | TAG 51:1 (16:0-17:0-18:1) | 0.14 |
| | TAG 52:6 (16:1-18:2-18:3) | 0.14 |
| | TAG 58:4 (18:1-18:2-22:1) | 0.13 |
| | TAG 53:4 (17:1-18:1-18:2) | 0.13 |
| | TAG 48:5 (12:0-18:2-18:3) | 0.12 |
| | TAG 58:5 (18:1-18:2-22:2) | 0.11 |
| | DAG 36:4 (18:2-18:2) | 0.11 |
| | TAG 49:1 (15:0-16:0-18:1) | 0.11 |
| | DAG 36:2 (18:1-18:1) | 0.10 |
| | TAG 49:2 (15:0-16:0-18:2) | 0.10 |
| | TAG 58:2 (18:1-18:1-22:0) | 0.10 |
| | TAG 58:3 (18:1-18:2-22:0) | 0.09 |
| | TAG 56:1 (16:0-18:1-22:0) | 0.08 |
| | DAG 34:0 (16:0-18:0) | 0.07 |
| | TAG 47:1 (12:0-17:0-18:1) | 0.07 |
| | DAG 32:0 (16:0-16:0) | 0.07 |
| | DAG 32:1 (14:0-18:1) | 0.06 |
| | TAG 47:2 (12:0-17:0-18:2) | 0.06 |
| | TAG 45:1 (12:0-15:0-18:1) | 0.06 |
| | TAG 42:3 (12:0-12:0-18:3) | 0.06 |
| | TAG 51:4 (15:0-18:2-18:2) | 0.06 |
| | DAG 32:2 (14:0-18:2) | 0.05 |
| | TAG 53:1 (17:0-18:0-18:1) | 0.05 |
| | TAG 58:1 (18:0-18:1-22:0) | 0.05 |
| | DAG 28:0 (12:0-16:0) | 0.05 |

(continued)

| Category | Glyceride type | Breast milk weight (%) |
|---|---|---|
|  | DAG 34:3 (16:1-18:2) | 0.04 |
|  | DAG 36:1 (18:0-18:1) | 0.04 |
|  | DAG 30:0 (14:0-16:0) | 0.04 |
|  | DAG 30:2 (12:0-18:2) | 0.04 |
|  | TAG 45:2 (12:0-15:0-18:2) | 0.04 |
|  | DAG 30:1 (12:0-18:1) | 0.04 |
|  | TAG 54:0 (16:0-18:0-20:0) | 0.04 |
|  | Total | 100.00 |

That is, the subsequent analysis on other dairy samples used the contents of characteristic glyerides in breast milk samples mentioned above as evaluation benchmarks.

However, it should be noted that due to differences in dietary and lifestyle habits in different regions, the compositions and contents of glycerides in breast milk of healthy lying-in women are different. Therefore, for those skilled in the art, the sources of breast milk samples can be selected according to actual needs, and the measurement results of characteristic glycerides in breast milk samples change accordingly.

Example 2

The scores of each dairy sample were evaluated by using the contents of characteristic glycerides in the breast milk sample obtained in Example 1 as evaluation benchmarks.

The score G was calculated according to the following formula:

$$G = 100 - \Sigma\left(100 \times \frac{H_i}{H_t} \times \frac{|H_{ih/l} - A_i|}{A_i}\right);$$

in the formula,
$$H_{ih/l} = \begin{cases} 1.3H_i, & A_i > 1.3H_i \\ A_i, & 1.3H_i \geq A_i \geq 0.7H_i \\ 0.7H_i, & A_i < 0.7H_i \end{cases}$$
; wherein, $H_i$ is the content of characteristic glyceride i in breast milk, $H_t$ is the content of total glycerides in breast milk, and $A_i$ is the content of the characteristic glyceride i in the sample. $A_i$ was measured by using the method that was the same as that for breast samples in Example 1. Cow milk based infant formula (CMIF), sheep milk based infant formula (SMIF), soy based formula (SIF), cow milk (CM), sheep milk (SM), camel milk (CaM), buffalo milk (BM), donkey milk (DM), horse milk (HoM), corn soybean oil (CSO), corn soybean sunflower seed oil (CSSO) and corn soybean sunflower seed coconut oil (CSSCO) were measured and scored by using the above evaluation method.

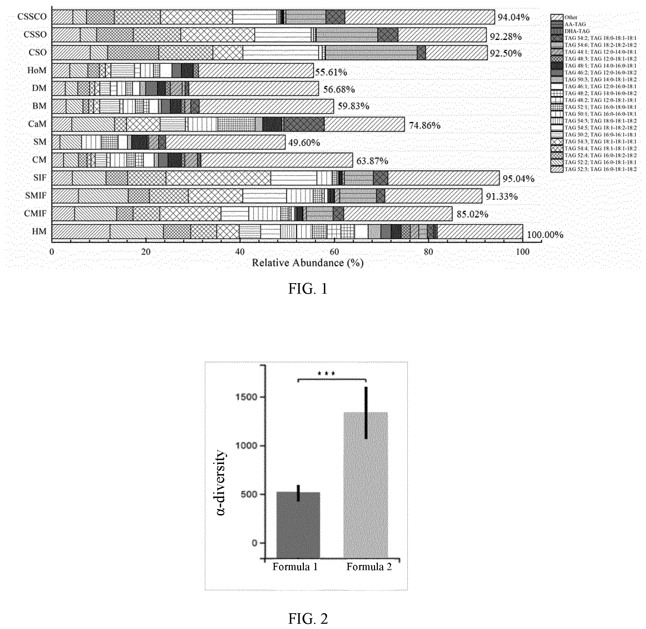

The contents of the same characteristic glycerides in the above various dairy samples were measured based on breast milk samples in Example 1 as a standard are shown in FIG. 1.

The calculated scores are shown in Table 3.

Table 3 Scores of each dairy sample

| Dairy samples | Score (G) |
|---|---|
| CMIF | 42.05 |
| SMIF | 41.57 |
| SIF | 19.07 |
| CM | 28.82 |

(continued)

| Dairy samples | Score (G) |
|---|---|
| SM | 16.13 |
| CaM | 26.20 |
| BM | 38.37 |
| DM | 36.79 |
| HoM | 36.33 |
| CSO | 12.00 |
| CSSO | 7.13 |
| CSSCO | 7.89 |

It can be seen from results of FIG. 1 and Table 3 that from the perspective of glycerides, there are significant differences between the existing infant formulas and breast milk in terms of composition and content deviation degree.

Example 3

In this example, actual samples were scored by using the method in Example 2, and the scores were verified from the perspective of nutrition.

The dairy samples in this example were ordinary infant formula (formula 1) and improved formula (formula 2).

The scores of the formula 1 and the formula 2 calculated by using the method in Example 2 were 37.63 and 47.23, respectively.

Nutrition verification was performed by using an animal experiment. 5 pregnant British white binary hybrid sows with similar maternal ages were selected. SPF piglets (n=20) born naturally were naturally delivered SPF sows. Healthy piglets with similar weight and length were selected, and breastfed uniformly within 3 days after birth to provide sufficient maternal immunity and nutrition for the piglets. Breastfeeding was conducted for 3 days prior to the start of the experiment, and then the feeding experiment was officially conducted until 21 days. After the formal experiment began, the experiment was randomly divided into three groups: breast milk group, formula group 1 and formula group 2. The piglets and sows in breast milk group were managed in the same pigsty and continued breastfeeding until 21 days of weaning; the formula groups were fed with reconstituted milk made of milk powder and water by using feeding bottles (later in a feeding trough) until 21 days. During this period, piglets were separated from sows and managed separately in cages. The samples were collected on the 21d after delivery.

The method for collecting serum was as follows: blood was collected in the morning under the condition of fasting after feeding for 21 days. Non-anticoagulant blood collection vessels were used to collect 3 mL of blood from the anterior vena cava, the blood stood for 1 h and then centrifuged for 20 min at 4 °C at 3000 rpm to obtain supernatant which was serum, and subsequently the serum was stored at -20 °C for immune index determination.

The collection method of feces samples: about 10 g of feces was collected from piglets after feeding for 21 days, quickly frozen in liquid nitrogen, transported with dry ice, and then stored in a refrigerator at a low temperature of -80 °C for later use.

High-throughput sequencing was carried out on 16S rRNA genes of intestinal flora. Genomic DNA was extracted from 100 mg of feces sample by using QIAamp Rapid Fecal DNA Mini Kit (Qiagen, GmbH, Hilden, German) according to reagent kit operating instructions. Then, 16S rRNA genes in V3-V4 regions were subjected to PCR amplification by using 10 ng of DNA as a template, 30 $\mu$L of total reaction system was as follows: 15 $\mu$L of high fidelity PCR reaction mixed solution (New England Biolabs), and 0.2 $\mu$M forward and reverse primers (314F-805R). PCR conditions were as follows: 95 °C, 3 min; 25 cycles in 30 s: 95 °C, 30 s; 30 s, 55 °C, 30 s, 72 °C. According to operating instructions, a sequencing library was generated by using NEB Ultra DNA preparation kit (NEB, USA). Amplicon sequencing was carried out by using PE 2$\times$250 bp HiSeq 2500 (Illumina, USA).

The merging, quality screening and demultiplex of raw offline sequencing data were carried out using QIIME 1.9.0 (Caporaso et al., 2010). The sequences were clustered, and the sequences with 97% similarity were classified into operational taxonomic units (OTU), then the OTU sequences were subjected to species annotation according to Greengenes 16S rRNA gene database (Version 13.5) (DeSantis et al., 2006), and classified into taxonomic levels from phylum to genus. Downstream data analysis was carried out with R language, including $\alpha$-diversity. $\beta$-diversity and other analysis were carried out using Bray-Curtis distance or UniFrac distance. The results are shown in FIGs. 2-4.

It can be seen from FIGs. 2-4 that compared with formula 1, formula 2 can significantly increase the diversity of intestinal bacteria of piglets so that the compositions of the intestinal bacteria are closer to those of breast milk, and greatly increases the level of immune factor IgA.

Nutrition verification was carried out using clinical experiments. Healthy full-term newborns were recruited under the support of hospitals, their legal guardians needed to carefully read the informed consent form and inform the researchers of their decision to participate in the study. Volunteers were randomly divided into 3 groups, including breast milk group, formula group 1 and formula group 2, respectively. During the study, the breast milk group adhered to exclusive breastfeeding, while the formula groups adhered to feeding with the given formula and were randomly divided into formula group 1 or formula group 2. During the study, other brands of milk powders were not allowed. Samples were respectively collected at 0-5 d, 1 month old, 2 months old, 3 months old, 4 months old, 5 months old, and 6 months old.

Evaluation of growth and development:
Physical development (body length, weight, head circumference, dental caries and skin) of infants were examined each month; at 3 months old and 6 months old, neuropsychological development was evaluated through ASQ scale.

Evaluation of intestinal micro-ecological environment health

Meconium from infants as well as feces from 1 month old, 2 months old, 3 months old, 4 months old, 5 months old and 6 months old was collected. The intestinal micro-ecological environment was reflected through the analysis of the microbial flora in the feces. For the breast milk group, the feces of lactating mothers were collected at the corresponding time points to investigate the influence of lactating mothers on the colonization of infant intestinal flora.

Infants aged 0-6 months were fed with native breast milk, formula 1 and formula 2 respectively, and the influences of different feeding methods on growth and development, immune functions, cognitive ability, intestinal flora and the like of infants and young children were monitored.

By measuring the heights and weights of infants aged 0-6 months, the physical development and nutrition of infants with different feeding methods were evaluated by using a Z-value method. The results are shown in Table 4.

Table 4 Comparison of WAZ values between infants aged 0-6 months with different feeding methods

| Month of age | Breast milk | Formula 1 | Formula 2 | F | P |
|---|---|---|---|---|---|
| 0 | $0.26\pm0.85$ | $-0.19\pm1.13$ | $-0.25\pm0.93$ | 5.871 | 0.003** |
| 1 | $0.76\pm0.94$ | $0.26\pm1.13$ | $0.48\pm1.44$ | 3.202 | 0.043* |
| 2 | $0.81\pm0.98$ | $0.28\pm1.09$ | $0.51\pm1.19$ | 4.449 | 0.013* |
| 3 | $0.78\pm0.97$ | $0.56\pm1.02$ | $0.68\pm1.13$ | 0.837 | 0.435 |
| 4 | $0.76\pm1.00$ | $0.65\pm0.98$ | $0.78\pm1.04$ | 0.368 | 0.693 |
| 5 | $0.72\pm1.09$ | $0.69\pm1.07$ | $0.83\pm1.08$ | 0.291 | 0.747 |
| 6 | $0.77\pm0.97$ | $0.56\pm1.39$ | $0.72\pm1.42$ | 0.405 | 0.668 |

It can be seen from Table 4 that at each month of age, the WAZ values of infants in each group are greater than -2, indicating that there are no low-weight infants in the entire process. Although the overall nutritional status of the two formula groups at birth is lower than that of breast milk group, after feeding for a period of time, the difference between the formula group 2 and the breast milk group gradually decreases and disappears after 3 months. It can be seen that the feeding effect of the formula group 2 is significantly better than that of formula group 1, and closer to that of breast milk.

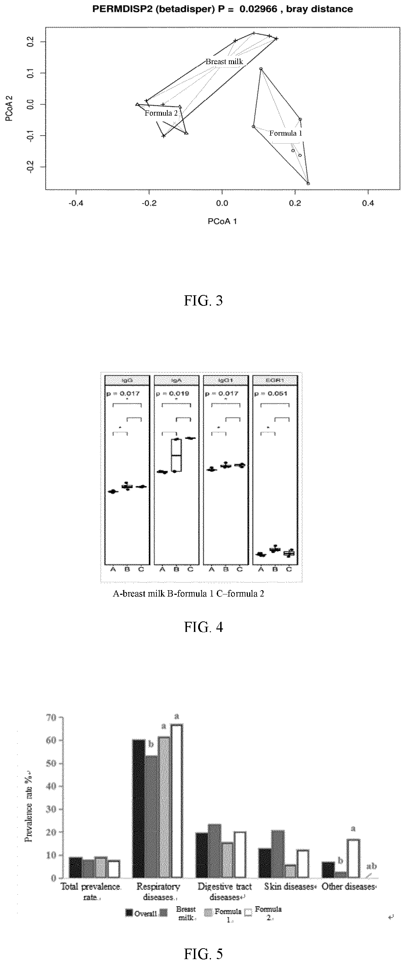

The prevalence rates of infants aged 0-6 months in the above groups are shown in FIG. 5. It can be seen from FIG. 5 that the overall prevalence rate of infants aged 0-6 months is below 10 %, the diseases suffered mainly include respiratory diseases accounting for more than 60 %. Different feeding methods have certain impact on the prevalence rate. Although there is no significant difference among the overall prevalence rates of the three groups, the overall prevalence rate of infants in formula group 1 is higher than that in breast milk group and formula group 2, which is especially manifested as uncommon other diseases; it is indicated that formula 2 with a higher score is closer to the effect of breastfeeding.

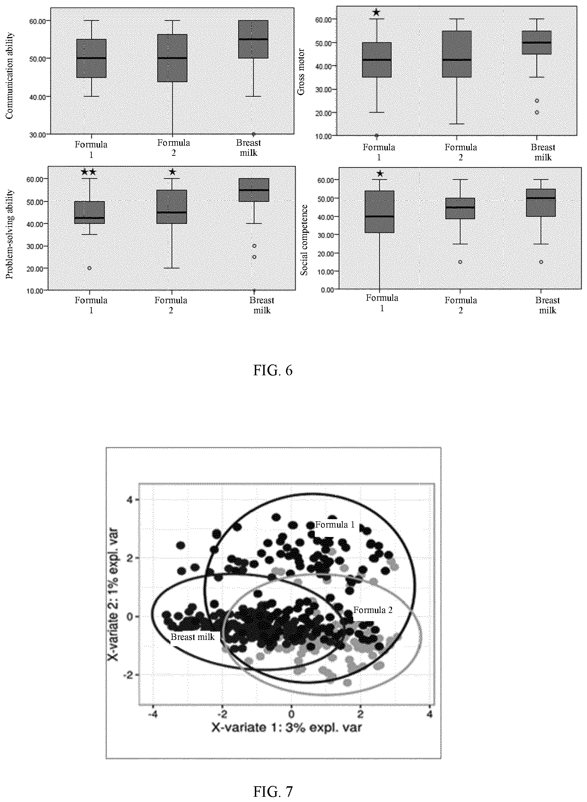

The neuropsychological development of infants aged 4 and 6 months in the above three groups was evaluated by using ASQ neuropsychological Assessment Scale, and the two results were synthesized for statistical analysis. The results are shown in FIG. 6. It can be seen from FIG. 6 that in terms of gross motor, social competence, problem-solving ability and overall score, formula group 2 is higher than formula group 1, and closer to breast milk group. The above result indicates that formula 2 with a higher score is significantly superior to formula 1 with a lower score in the aspect of promoting the neuropsychological development of infants, and has a feeding effect that is closer to that of breast milk.

The composition of intestinal flora of infants in the above groups was determined. The results are shown in FIG. 7. It can be seen from FIG. 7 that the composition of intestinal flora of infants in formula group 2 is closer to that in breast milk group and superior to that in formula group 1.

In conclusion, after various experiments, formula group 2 with a higher score is superior to formula group 1 with a lower score in the aspects of promoting the development of infants, enhancing the resistance of infants, promoting the neuropsychological development of infants and promoting the intestinal microbial growth of infants, and is closer to breast milk group. Therefore, the method for evaluating dairy products based on lipid components provided in the present application can objectively evaluate the nutritional level of dairy products or raw and auxiliary materials in a molecular level, and has certain guiding significance for subsequent simulation of breast milk in a molecular level.

The above descriptions provide a detailed description of the preferred embodiments of the present application. However, the present application is not limited to the specific details of the aforementioned embodiments. Within the scope of the technical concept of the present application, multiple simple modifications can be made to the technical solution of the present application, all of which fall within the scope of protection of the present application.

In addition, the various implementation methods of the present application can also be combined arbitrarily, as long as they do not violate the ideas of the present application, they should also be considered as the content disclosed in the present application.

**Industrial applicability**

The present application relates to a method for evaluating dairy products based on lipid components. The present application evaluates infant formula and raw and auxiliary materials in multiple dimensions from two aspects of composition and content of glyceride, thereby objectively evaluating the nutritional level of dairy products or raw and auxiliary materials in a molecular level, which has certain guiding significance for subsequent simulation of breast milk.

**Claims**

1. A method for evaluating dairy products based on lipid components, the method being carried out according to the following steps:

   (1) Establishing local breast milk glyceride database;
   (2) Collecting and pretreating to-be-evaluated dairy product samples;
   (3) Measuring the contents of characteristic glycerides in the to-be-evaluated dairy product samples; and
   (4) Calculating the scores G of the to-be-evaluated dairy product samples based on the following formula:

$$G = 100 - \Sigma\left(100 \times \frac{H_i}{H_t} \times \frac{|H_{ih/1} - A_i|}{A_i}\right);$$

   In the formula,
$$H_{ih/1} = \begin{cases} 1.3H_i, & A_i > 1.3H_i \\ A_i, & 1.3H_i \geq A_i \geq 0.7H_i \\ 0.7H_i, & A_i < 0.7H_i \end{cases}$$
; Wherein, $H_i$ is the content of characteristic glyceride i in breast milk, $H_t$ is the content of total glycerides in breast milk, and $A_i$ is the content of the characteristic glyceride i in the sample.

2. The method for evaluating dairy products according to claim 1, wherein in step (2), the pretreating is carried out according to the following steps:
   diluting a sample with a redissolving solution so that the concentration of the sample is consistent with that of breast milk, adding an internal standard to the sample, then adding ultrapure water, methanol and dichloromethane, mixing the above materials, and then adding ultrapure water and dichloromethane, centrifuging the mixed solution to separate an organic phase from an aqueous phase, adding ultrapure water, methanol and dichloromethane into the organic phase, evenly mixing, then centrifuging the mixed solution to separate an organic phase from an aqueous phase, merging the aqueous phases obtained after the two centrifugations, adding dichloromethane, mixing and centrifuging to separate an organic phase from an aqueous phase; and merging the organic phases obtained after the last two centrifugations, blowing the organic phases to be dried with nitrogen, and then redissolving the dried organic phases with the redissolving solution.

3. The method for evaluating dairy products according to claim 2, wherein the redissolving solution used in the pretreating is a methanol-dichloromethane solution containing 0.5-2 mM ammonium acetate, and a volume ratio of methanol to dichloromethane in the redissolving solution is 1:1-2; preferably, the redissolving solution is a methanol-dichloromethane solution containing 1 mM ammonium acetate, and a volume ratio of methanol to dichloromethane in the redissolving solution is 1:1.

4. The method for evaluating dairy products according to claim 2, wherein the internal standard is a d5-TAG54:3 standard product with a concentration of 3000-5000 mg/L, preferably 4000 mg/L.

5. The method for evaluating dairy products according to claim 1, wherein in step (3), a method for measuring the contents of characteristic glycerides is an ultra-high performance liquid chromatography tandem quadrupole time-of-flight mass spectrometry.

6. The method for evaluating dairy products according to claim 5, wherein the method for the ultra-high performance liquid chromatography is as follows: a chromatographic column is Phenomenex, Kinetex 2.6 $\mu$m C18 100 Å 50 × 3 mm, with an injection volume of 2 $\mu$L, a flow rate of 0.8 mL/min and a column temperature of 30 °C; mobile phase A is a water/methanol/acetonitrile (1:1:1, v/v/v) solution of 5 mM ammonium acetate, and mobile phase B is an isopropanol solution of 5 mM ammonium ethanol; an elution gradient is:

| Glyceride (time, min) | 0.0 | 5.0 | 8.0 | 8.01 | 10.0 |
|---|---|---|---|---|---|
| A | 50% | 2% | 2% | 50% | 50% |
| B | 50% | 98% | 98% | 50% | 50% |

7. The method for evaluating dairy products according to claim 5, wherein the conditions of the quadrupole time-of-flight mass spectrometry are as follows: under the condition of a positive pole, a spray voltage of an ion source is 5500 V, a gas pressure of the ion source is 60 psi, a temperature of the ion source is 650 °C, a pressure of a curtain gas is 35 psi, a de-clustering voltage is 80 V, a collision energy is 10 V, and a scanning range is 50-1300 Da.

8. The method for evaluating dairy products according to claim 1, wherein in step (3), the characteristic glycerides comprise TAG52:3 (16:0-18:1-18:2), TAG52:2 (16:0-18:1-18:1), TAG52:4 (16:0-18:2-18:2), TAG54:4 (18:1-18:1-18:2), TAG54:3 (18:1-18:1-18:1), TAG50:2 (16:0-16:1-18:1), TAG54:5 (18:1-18:2-18:2), TAG54:3 (18:0-18:1-18:2), TAG50:1 (16:0-16:0-18:1), TAG52:1 (16:0-18:0-18:1), TAG48:2 (12:0-18:1-18:1), TAG48:2 (14:0-16:0-18:2), TAG46:1 (12:0-16:0-18:1), TAG50:3 (14:0-18:1-18:2), TAG46:2 (12:0-16:0-18:2), TAG48:1 (14:0-16:0-18:1), TAG48:3 (12:0-18:1-18:2), TAG44:1 (12:0-14:0-18:1), TAG54:6 (18:2-18:2-18:2), and TAG54:2 (18:0-18:1-18:1).

9. The method for evaluating dairy products according to claim 8, wherein the characteristic glycerides further comprise TAG58:8 (18:1-18:1-22:6), TAG58:9 (18:1-18:2-22:6), TAG58:10 (18:2-18:2-22:6), TAG56:7 (18:1-18:2-20:4), and TAG56:8 (18:2-18:2-20:4).

10. The method for evaluating dairy products according to claim 9, wherein the characteristic glycerides further comprise TAG 44:2 (12:0-14:0-18:2), TAG 52:5 (16:0-18:2-18:3), TAG 50:4 (14:0-18:2-18:2), TAG 44:0 (12:0-14:0-18:0), TAG 42:0 (12:0-14:0-16:0), TAG 48:4 (12:0-18:2-18:2), TAG 42:1 (12:0-12:0-18:1), TAG 40:0 (12:0-14:0-14:0), TAG 46:0 (12:0-16:0-18:0), TAG 46:3 (12:0-16:1-18:2), TAG 48:0 (14:0-16:0-18:0), TAG 56:5 (18:1-18:2-20:2), TAG 56:6 (18:1-18:2-20:3), TAG 50:0 (16:0-16:0-18:0), TAG 42:2 (12:0-12:0-18:2), TAG 38:0 (12:0-12:0-14:0), TAG 56:4 (18:1-18:2-20:1), TAG 56:3 (18:1-18:1-20:1), TAG 54:7 (18:2-18:2-18:3), TAG 40:1 (12:0-12:0-16:1), TAG 54:1 (18:0-18:0-18:1), TAG 54:1 (16:0-18:1-20:0), TAG 36:0 (12:0-12:0-12:0), DAG 34:2 (16:0-18:2), TAG 56:2 (16:0-18:1-22:1), TAG 52:0 (16:0-18:0-18:0), DAG 34:1 (16:0-18:1), TAG 56:3 (18:1-18:2-20:0), TAG 51:2 (16:0-17:1-18:1), TAG 53:2 (17:0-18:1-18:1), TAG 50:5 (14:0-18:2-18:3), TAG 58:6 (18:1-18:1-22:4), TAG 53:3 (17:0-18:1-18:2), TAG 58:7 (18:1-18:2-22:4), TAG 44:3 (12:0-14:0-18:3), DAG 36:3 (18:1-18:2), TAG 51:3 (15:0-18:1-18:2), TAG 51:1 (16:0-17:0-18:1), TAG 52:6 (16:1-18:2-18:3), TAG 58:4 (18:1-18:2-22:1), TAG 53:4 (17:1-18:1-18:2), TAG 48:5 (12:0-18:2-18:3), TAG 58:5 (18:1-18:2-22:2), DAG 36:4 (18:2-18:2), TAG 49:1 (15:0-16:0-18:1), DAG 36:2 (18:1-18:1), TAG 49:2 (15:0-16:0-18:2), TAG 58:2 (18:1-18:1-22:0), TAG 58:3 (18:1-18:2-22:0), TAG 56:1 (16:0-18:1-22:0), DAG 34:0 (16:0-18:0), TAG 47:1 (12:0-17:0-18:1), DAG 32:0 (16:0-16:0), DAG 32:1 (14:0-18:1), TAG 47:2 (12:0-17:0-18:2), TAG 45:1 (12:0-15:0-18:1), TAG 42:3 (12:0-12:0-18:3), TAG 51:4 (15:0-18:2-18:2), DAG 32:2 (14:0-18:2), TAG 53:1 (17:0-18:0-18:1), TAG 58:1 (18:0-18:1-22:0), DAG 28:0 (12:0-16:0), DAG 34:3 (16:1-18:2), DAG 36:1 (18:0-18:1), DAG 30:0 (14:0-16:0), DAG 30:2 (12:0-18:2), TAG 45:2 (12:0-15:0-18:2) and DAG 30:1 (12:0-18:1), and TAG 54:0 (16:0-18:0-20:0).

FIG. 1

FIG. 2

**PERMDISP2 (betadisper) P = 0.02966 , bray distance**

FIG. 3

A-breast milk B-formula 1 C–formula 2

FIG. 4

FIG. 5

FIG. 6

FIG. 7

## INTERNATIONAL SEARCH REPORT

| | International application No. |
|---|---|
| | **PCT/CN2023/089177** |

**A. CLASSIFICATION OF SUBJECT MATTER**

G01N33/04(2006.01)i

According to International Patent Classification (IPC) or to both national classification and IPC

**B. FIELDS SEARCHED**

Minimum documentation searched (classification system followed by classification symbols)

IPC: G01

Documentation searched other than minimum documentation to the extent that such documents are included in the fields searched

Electronic data base consulted during the international search (name of data base and, where practicable, search terms used)

CNABS, CNTXT, VEN, CNKI: 乳类, 奶类, 产品, 制品, 母乳, 甘油酯, 甘油, 酯, milk, product, breast milk, glyceride

**C. DOCUMENTS CONSIDERED TO BE RELEVANT**

| Category* | Citation of document, with indication, where appropriate, of the relevant passages | Relevant to claim No. |
|---|---|---|
| PX | CN 115267098 A (BEIJING SANYUAN FOODS CO., LTD.) 01 November 2022 (2022-11-01) <br> description, paragraphs 7-24 | 1-10 |
| A | CN 111999407 A (BEIJING SANYUAN FOODS CO., LTD.) 27 November 2020 (2020-11-27) <br> description, paragraphs 9-26 | 1-10 |
| A | CN 112858558 A (SHAANXI UNIVERSITY OF SCIENCE & TECHNOLOGY) 28 May 2021 (2021-05-28) <br> entire document | 1-10 |
| A | CN 104165955 A (ZHEJIANG UNIVERSITY et al.) 26 November 2014 (2014-11-26) <br> entire document | 1-10 |
| A | CN 107549313 A (INSTITUTE OF FOOD SCIENCE AND TECHNOLOGY, CHINESE ACADEMY OF AGRICULTURAL SCIENCES) 09 January 2018 (2018-01-09) <br> entire document | 1-10 |
| A | CN 111665298 A (OCEAN UNIVERSITY OF CHINA) 15 September 2020 (2020-09-15) <br> entire document | 1-10 |

☑ Further documents are listed in the continuation of Box C.    ☑ See patent family annex.

| | |
|---|---|
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **01 August 2023** | **04 August 2023** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/089177**

### C.    DOCUMENTS CONSIDERED TO BE RELEVANT

| Category* | Citation of document, with indication, where appropriate, of the relevant passages | Relevant to claim No. |
|---|---|---|
| A | CN 104186701 A (JIANGNAN UNIVERSITY) 10 December 2014 (2014-12-10)<br>entire document | 1-10 |
| A | US 2009197340 A1 (VALOREX SA) 06 August 2009 (2009-08-06)<br>entire document | 1-10 |
| A | US 2022099647 A1 (CANALE STEPHANIE) 31 March 2022 (2022-03-31)<br>entire document | 1-10 |
| A | 李子豪等 (LI, Zihao et al.). "乳及乳制品中磷脂检测技术研究进展 (Research progress of detection technique of phospholipids in milk and milk products)"<br>食品安全质量检测学报 (Journal of Food Safety and Quality),<br>Vol. 13, No. 7, 30 April 2022 (2022-04-30), 2051-2058<br>the third section, detection method of phospholipids | 1-10 |

Form PCT/ISA/210 (second sheet) (July 2022)

**INTERNATIONAL SEARCH REPORT**
Information on patent family members

International application No.

**PCT/CN2023/089177**

| Patent document cited in search report | | | Publication date (day/month/year) | Patent family member(s) | | | Publication date (day/month/year) |
|---|---|---|---|---|---|---|---|
| CN | 115267098 | A | 01 November 2022 | None | | | |
| CN | 111999407 | A | 27 November 2020 | None | | | |
| CN | 112858558 | A | 28 May 2021 | None | | | |
| CN | 104165955 | A | 26 November 2014 | None | | | |
| CN | 107549313 | A | 09 January 2018 | None | | | |
| CN | 111665298 | A | 15 September 2020 | None | | | |
| CN | 104186701 | A | 10 December 2014 | None | | | |
| US | 2009197340 | A1 | 06 August 2009 | CA | 2652543 | A1 | 13 December 2007 |
| | | | | CA | 2652543 | C | 19 June 2012 |
| | | | | BRPI | 0711488 | A2 | 22 February 2012 |
| | | | | BRPI | 0711488 | B1 | 06 March 2018 |
| | | | | PL | 2032966 | T3 | 31 May 2013 |
| | | | | US | 7803625 | B2 | 28 September 2010 |
| | | | | WO | 2007141416 | A1 | 13 December 2007 |
| | | | | EP | 2032966 | A1 | 11 March 2009 |
| | | | | EP | 2032966 | B1 | 19 December 2012 |
| | | | | ES | 2401197 | T3 | 17 April 2013 |
| US | 2022099647 | A1 | 31 March 2022 | None | | | |

Form PCT/ISA/210 (patent family annex) (July 2022)

**EP 4 339 610 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210902419